# EUROPEAN PATENT APPLICATION

(11) **EP 3 095 516 A1**
(43) Date of publication of application: **23.11.2016**
(21) Application number: 16466008.6
(22) Date of filing: 16.05.2016
(51) Int. Cl.: B01J 21/06, B01J 23/80, B01J 35/00, B01J 37/02, B01J 35/02, A61L 9/20, B01D 53/86, C01G 23/04, C01G 23/047, C09D 1/00, C03C 17/245, C03C 17/25, F24F 3/16, B01D 53/00, B01D 53/34

(54) **PHOTOCATALYTIC FILTER DEVICE FOR AIR PURIFICATION, BASED ON TITANIUM OXIDE COMBINED WITH COLLOIDAL COPPER AND ZINC**

(30) Priority: 18.05.2015 CZ 20150329
(71) Applicant: Hersálek Milos, 463 43 Svetlá pod Jestedem (CZ)
(72) Inventor: Hersálek Milos, 463 43 Svetlá pod Jestedem (CZ)
(74) Representative: Novotny, Karel

(57) **Abstract**

The invention relates to a photocatalytic filter equipment designed for filtering air, the chamber (2) of which is equipped with inlet (8) and outlet (9) openings for the flow of filtered air and metal plates (3) which are arraged orthogonal to UV tubes (4). The sidewalls of the chamber and the metal plates, after being pre-treated with a copolymer, are coated with titanium oxide modified by colloidal zinc and copper.

This filter device can be used in sequence with for example an air conditioning system.

## Description

### Technical Field of the Invention

The invention pertains to Nano Photocatalytic Filter Equipment designed for filtering air from nitrogen oxide, formaldehyde, aerosol airborne dust, bacteria, fungi, viruses, yeast cells in sizes smaller than 10 µm, intended as a replacement of any solid filters in any air-conditioning system, such as air purifiers, heat recovery ventilation systems, refrigerating and air-conditioning units, air-conditioning pipes.

### State-of-the-art

All the above mentioned devices use various kinds of solid filters for air cleaning, which, however, are prone to wear in a relatively short period of time and, contrariwise, they are the cause of multiplication of viruses, bacteria, fungi and yeast cells. These filters are only able to capture particles up to 100 µm.

The present invention is filtering equipment which is able to capture harmful substances to a greater extent while achieving a long service life of the equipment without changing the filter.

### Subject Matter of the Invention

According to the invention, the nano photocatalytic filter equipment for air filtration, comprising a chamber with inlet and outlet openings, consists in plates of metal or another non-porous material arranged inside a chamber with transverse UV tubes (4), the plates being coated with a photocatalytic coat. The plates are treated with copolymer, the photocatalytic coat contains zinc- and copper-modified titanium oxide TiO. The filter equipment can be connected between an air-conditioning system (7) and workspace (1)

### Survey of Figures in Drawings

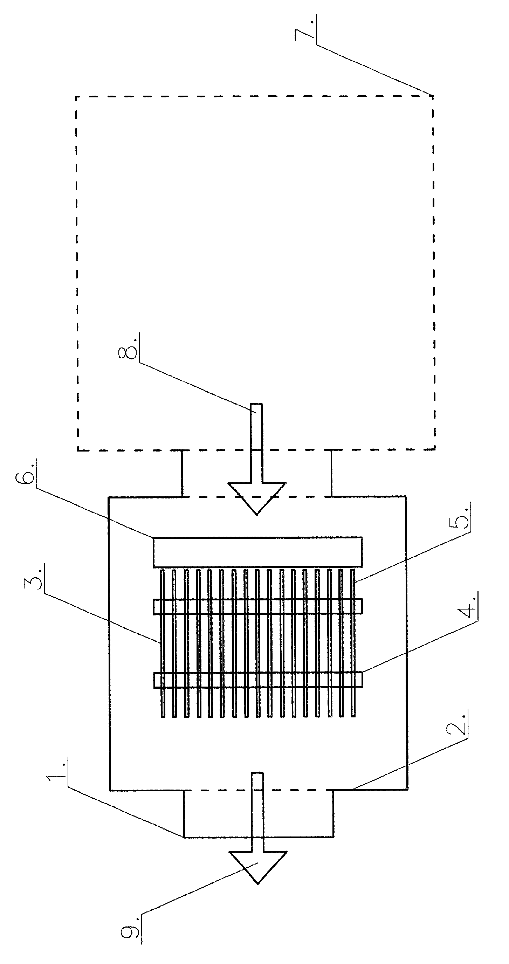

The accompanying figure shows the filter equipment of the present invention.

### Example of an Embodiment of the Invention

The nano photocatalytic air purification filter equipment is connected to the output of an air-conditioning system 7 and consists of at least one chamber 2 fitted with plates 3 among which air exiting from the air-conditioning system is passing through 7. There are UV tubes 4 passing across these plates.

The plates 3 are made of non-porous material, coated with a photocatalytic layer 5 of titanium oxide optionally modified by colloidal zinc and copper. After being coated with titanium oxide, the plates are heat-treated. Before being coated with titanium oxide, the surface of the plates is treated with copolymer. The surface is thereby fixed on the non-porous surface and is resistant both to abrasion and water. Clean air is conveyed by means of a fan 6. The air from the air-conditioning system 7 flows into the inlet opening 8, passes through the chamber 2 and filtered air exits through the outlet opening 9 into workspace 1.

The nano photocatalytic filter equipment uses a known method of chemical decomposition of nanometre-sized substances in the presence of a photocatalyst and UV radiation, known as photocatalysis. If material having photocatalytic properties, i.e. with a photocatalyst, is exposed to UV radiation of a suitable wavelength, its surface is activated and a characteristic reaction is triggered in which a primarily generated free pair electron-hole and hydroxyl radicals, formed by the contact of the excited molecule of the photocatalyst with water vapour, decompose the present organic and inorganic substances. Substances degradable in photocatalysis include nitrogen oxides (NOx), sulphur oxides (S02), carbon oxide (CO), ozone (03), ammonia (NH3), hydrogen sulphide (H2S), chlorinated hydrocarbons, aromatic hydrocarbons (benzene, phenol, toluene, ethylbenzene), pesticides as well as bacteria, viruses, picornaviruses, fungi and microdust. The final products are stable compounds.

Nanocrystalline titanium oxide Ti02 modified by colloidal zinc and copper is used as a catalyst. This catalyst is applied to a non-porous material which had been previously treated with copolymer, stabilized at an elevated temperature. The copolymer ensures the adhesion of titanium oxide TiO2 to the metal surface of the plates.

The nano photocatalytic filter equipment is formed by a large number of plates, creating a large surface with fluorescent tubes passing through, placed so as to illuminate each part of the catalyst-coated plate by UV radiation. The illuminated area is many times bigger than the actual volume of the filter equipment. The nano photocatalytic filter is always fixed at the air outlet from the air-conditioning equipment, eliminating the need for fitting the air-conditioning system with solid filters and regular replacing thereof, and its efficiency is increased severalfold compared to the efficiency of the original filters.

### Industrial Applicability

The present invention is applicable in any air-conditioning system ensuring cleanliness of the air exiting from such systems and replaces all rapidly aging filters which are consequently a source of bacteria, viruses and fungi. The present invention may be used as a separate filter unit in closed buildings.

## Claims

1. The nano photocatalytic filter equipment for air filtration comprising a chamber (2) with inlet (8) and outlet (9) openings, where there are surfaces coated with a photocatalytic layer and an UV radiation source arranged in the chamber, **characterized in that** inside the chamber (2), there are arranged plates (3) of metal or another non-porous material with transverse UV tubes (4), where the plates (3) treated with copolymer are coated with a photocatalytic layer (5) containing zinc- and copper-modified titanium oxide TiO.

2. The nano photocatalytic filter equipment for air filtration recited in claim 1 wherein the equipment can be connected between an air-conditioning system (7) and workspace (1).
